Europäisches Patentamt

European Patent Office

Office européen des brevets

(11) Publication number: **0 344 371**
**A1**

(12) **EUROPEAN PATENT APPLICATION**

(21) Application number: 88304960.3

(22) Date of filing: 31.05.88

(51) Int. Cl.⁴: **C13K 1/02** , **C13K 13/00** ,
**C12P 19/14**

(43) Date of publication of application:
06.12.89 Bulletin 89/49

(84) Designated Contracting States:
AT BE CH DE ES FR GB IT LI NL SE

(71) Applicant: UOP INC.
25 East Algonquin Road
Des Plaines Illinois 60017-5017(US)

(72) Inventor: Arena, Blaise J.
1324 Jeanette
Des Plaines Illinois 60018(US)
Inventor: Allenza, Paul
1719 Forest Cove Drive
Des Plaines Illinois 60018(US)

(74) Representative: Brock, Peter William
URQUHART-DYKES & LORD 91 Wimpole
Street
London W1M 8AH(GB)

(54) Hydrolysis of corn kernel hulls to monosaccharides.

(57) Corn kernel hulls can be hydrolyzed to monosaccharides by a sequence of acid and enzymatic hydrolysis steps, without encountering the problems arising with lignocellulosic materials.

Acid hydrolysis at more elevated temperatures (80 to 110°C) leads to a liquid containing chiefly D-glucose, D-xylose and L-arabinose, and a solid which is converted chiefly into D-glucose on enzymatic hydrolysis.

Acid hydrolysis at a lower temperature (35 to 80°C) leads to a liquid containing chiefly D-xylose and L-arabinose, and a solid which is converted chiefly into D-glucose on enzymatic hydrolysis.

An initial digestion of the hulls with dilute base, followed by enzymatic hydrolysis forms a liquid (whose monosaccharide is chiefly D-glucose) and a solid portion, which on acid hydrolysis forms chiefly D-glucose, D-xylose and L-arabinose.

FIG.3

# HYDROLYSIS OF CORN KERNEL HULLS TO MONOSACCHARIDES

This invention concerns the production of selected monosaccharides by hydrolyzing corn kernel hulls, employing a sequence of hydrolysis steps.

Monosaccharides generally find broad use in commerce and, of the monosaccharides, glucose particularly finds varied usage. Glucose is used as the chief substrate in fermentation media for production of ethanol, is isomerized to afford fructose, which is broadly used as a sweetener, and is itself used as a sweetener, especially in confectionary products, although glucose is only perhaps three-fourths as sweet as sugar.

A major source of glucose is cellulose, a polysaccharide of cellobiose, which is the (1 → 4)-linked disaccharide of beta-D-glucose. In the United States, corn cobs are a major source of cellulose, for cobs are an abundant waste material resulting from one of the largest agricultural crops. The use of corn cobs is not without disadvantages, which arise chiefly from the presence of lignin. Corn cobs are within the class known as lignocellulosics, where cellulose is embedded in a matrix of amorphous lignin and hemicellulose. Pretreatment of lignocellulosics is necessary, in order to disrupt the lignin matrix so that cellulose becomes more available to subsequent hydrolytic agents, and a whole class of processes is directed towards removal of lignin from lignocellulosics. The disadvantages of delignifying pretreatments are increased by the relatively low value of lignin and the need to dispose of the chemical waste arising from the delignification process.

In the context of glucose production, it would be highly advantageous either to develop a more efficient, less costly delignification process, or to find an essentially lignin-free source of cellulose. Such a source would be desirably available in abundance and by products accompanying glucose production would be of distinct commercial value, so that the source could be efficiently used with a minimum of waste disposal problems. We have found such a source of cellulose in corn kernel hulls, a waste product of corn milling operations, which contain little or no lignin. Consequently, corn kernel hulls can be hydrolyzed in high yield, without any delignifying pretreatment, to afford a mixture which is mainly D-glucose, D-xylose, and L-arabinose. The latter two monosaccharides, which are pentoses, have independent utility as components of culture and fermentation media for some microorganisms, with D-xylose also being used in dyeing and tanning, so that virtually all the monosaccharides arising from hydrolysis have a commercial niche, a circumstance with important economic advantages.

Recognizing the advantages accruing from an abundant source of cellulose, which requires no delignification pretreatment to make cellulose available to hydrolytic agents, several variants on a theme of hydrolyzing corn kernel hulls to a mixture of monosaccharides have now been found.

In one embodiment, acid hydrolysis at elevated temperature followed by enzymatic hydrolysis affords a maximum yield of glucose and total monosaccharides.

A second embodiment employs acid hydrolysis at lower temperature, to afford a solution whose monosaccharides are chiefly those from hemicellulose, with subsequent enzymatic hydrolysis cleaving cellulose to liberate glucose.

A third embodiment features a very mild base pretreatment, followed by enzymatic hydrolysis of cellulose to afford a solution whose monosaccharide is virtually exclusively glucose, and further acid treatment then hydrolyzes the hemicellulose component.

Each of these embodiments exhibits particular advantages recommending its use, depending upon the marketplace and the needs of the processor, making the theme especially harmonious. Each embodiment also has unique features discovered during its development, which may be inherent in the use of corn kernel hulls as a feedstock.

The various embodiments of the invention will be further described with reference to the accompanying Drawing, in which:

Figure 1 is a flow scheme for hydrolysis of corn kernel hulls using in sequence strong acid at a temperature over 80°C, followed by enzymatic hydrolysis, with an optional separation stage after acid hydrolysis.

Figure 2 is a flow scheme for hydrolysis of corn kernel hulls using in sequence strong acid at a temperature under 80°C, followed by enzymatic hydrolysis, with an optional separation stage after acid hydrolysis.

Figure 3 is a flow scheme for hydrolysis of corn kernel hulls using a mild base pretreatment followed first by enzymatic hydrolysis, then by acid hydrolysis, with an optional separation stage after enzymatic hydrolysis.

This invention is concerned with the problem of developing a method of making D-glucose, D-xylose, and L-arabinose, from a readily abundant material without the need for any delignifying pretreatment. An embodiment comprises hydrolyzing corn kernel hulls with acid at elevated temperature with subsequent enzymatic hydrolysis of the hydrolysate. In a more specific embodiment, acid hydrolysis is conducted at a temperature from 80 to 110°, more especially from 85 to 110° C. In another embodiment, corn kernel hulls are hydrolyzed with acid at a temperature between 35 and 80° C, preferably less than 75° C, to afford a mixture of pentoses which may be separately recovered, followed by enzymatic hydrolysis to afford glucose from the unhydrolyzed cellulose. In yet another embodiment, a mild base pretreatment of corn kernel hulls is followed by enzymatic hydrolysis to afford glucose as the substantially exclusive monosaccharide, with subsequent acid hydrolysis of the hemicellulose and unreacted cellulose components.

The present invention arises from several discrete discoveries. The basic one, from the corn wet milling industry, is that corn kernel hulls contain little if any lignin. A typical analysis of corn kernel hulls shows about 20% starch, about 30% cellulose, about 30% hemicellulose, about 10% protein, and less than 5% lignin. Consequently, corn kernel hulls act differently from typical lignocellulosics, in not requiring delignification in order conveniently to hydrolyze the cellulose and hemicellulose components. Secondly, it has now been discovered that, in the acid hydrolysis of corn kernel hulls, the yield of glucose is quite temperature-dependent, whereas the yield of the pentose, D-xylose and L-arabinose, is relatively constant. This permits a degree of control of hydrolyzate content not heretofore appreciated. It has also been discovered that a relatively mild base pretreatment suffices to disrupt the crystallinity of cellulose in corn kernel hulls, to a degree where it is susceptible to enzymatic hydrolysis. The combination of two or more of these discoveries affords variants of hydrolytic processes which are described and claimed within.

One noteworthy feature of the present invention is its flexibility; each process scheme has options which permits the processor to take advantage of current market conditions. Another noteworthy feature of the present invention is that some of its options inherently lead to separation of the monosaccharide hydrolysis products into hexose and pentose streams, without necessitating a separate and distinct separations stage, other than simple filtration.

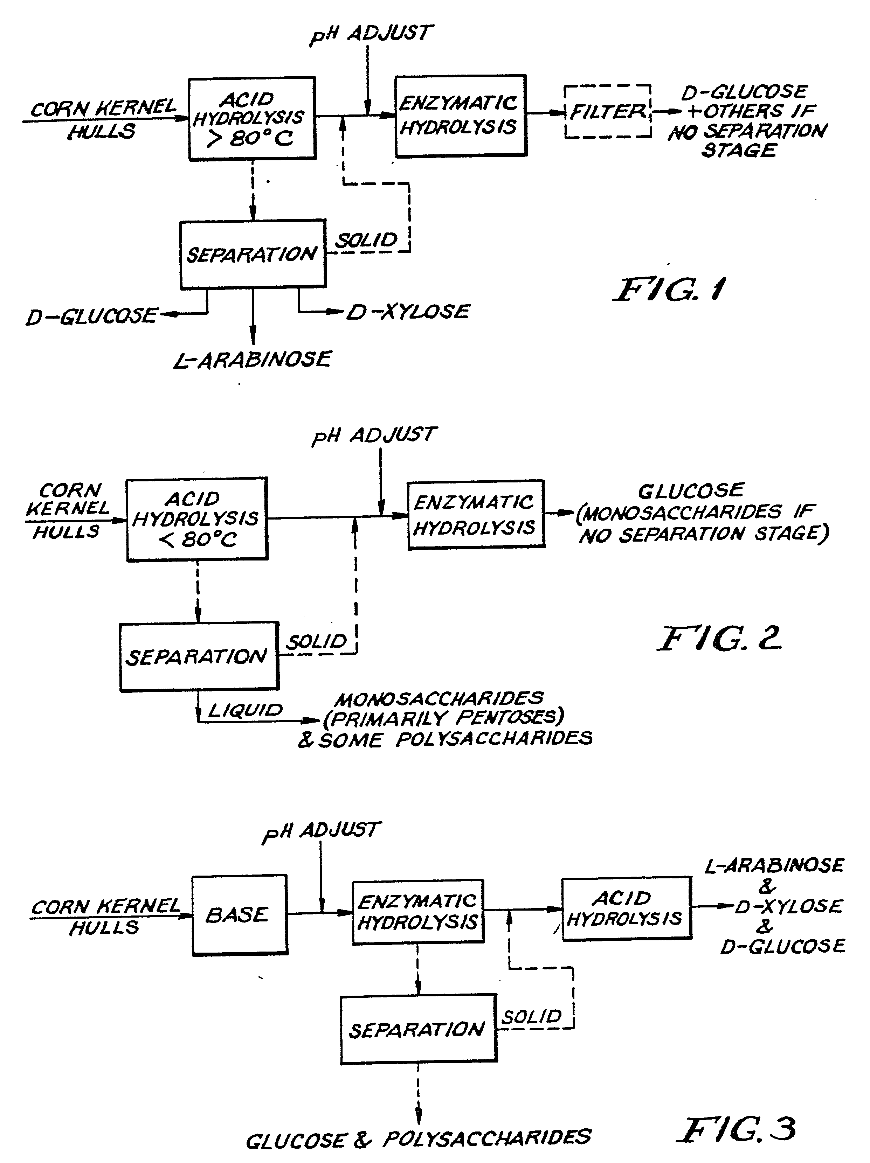

A flow scheme for the first embodiment is depicted in Figure 1. In this embodiment, corn kernel hulls are hydrolyzed by sequentially subjected the hulls to hydrolysis with a strong acid at a temperature in the range from 80 to 110° C, then subjecting the acid hydrolysate to hydrolysis with a cellulose-degrading enzyme, and recovering the resulting enzymatic hydrolysate. Among the strong acids which may be used in the initial acid hydrolysis are sulphuric acid, hydrochloric acid, phosphoric acid, hydrofluoric acid, trifluoroacetic acid, trichloroacetic acid, and so on. The particular nature and identity of the strong acid used is not important, so long as it is not an oxidizing acid under reaction conditions. Acid concentrations typically are in the range from 0.5 to 15% by weight, and more typically are in the range from 3 to 10% by weight.

Hydrolysis with strong acid is carried out between 80 and 110° C. The maximum temperature is dictated by the observation that, at temperatures in excess of 110° C, appreciable degradation of the hydrolyzate may occur. The minimum temperature is dictated by the observation that glucose yields are quite temperature-dependent, requiring inordinately long reaction times at lower temperatures to attain maximum glucose production. A temperature range between 95 and 105° C appears to be optimal.

At least a portion of the reaction product from acid hydrolysis is then treated with a cellulose-degrading enzyme to complete the hydrolysis of unreacted cellulose. The enzyme cellulose is commonly used at a temperature between 25 and 55° C, preferably between 35 and 50° C. Generally, the mixture is buffered to a pH between 3 and 6, more commonly in the range from 4.0 to 5.0. When enzymatic hydrolysis is complete the resulting hydrolysate is recovered and processed according to the needs of the manufacturer. Thus, glucose may be separated from the pentoses, the monosaccharides separated from all other components, or, perhaps, the entire hydrolysate can be used as such without further processing.

As shown in Figure 1, the reaction product from acid hydrolysis can be separated into a liquid portion and a solid portion. Separation may be by any convenient means, such as by membrane separation or by simple filtration. Separation by filtration is preferred, and where practiced it is important to not dry the filter cake before its subsequent use as described below. The monosaccharides of the liquid portion are chiefly D-glucose, D-xylose, and L-arabinose, and the liquid portion may be separately processed to isolate and purify one or more of the constituent monosaccharides. The solid portion obtained in separation is then subjected to the enzymatic hydrolysis. The hydrolyzate therefrom is recovered, and is found to contain chiefly D-glucose.

The solid separation step described above is preferred when the maximum total glucose yield is desired. That is, this procedure affords a greater total glucose yield that if no separation of liquid and solid from strong acid hydrolysis is practiced. This procedure also affords an enzymatic hydrolyzate which contains D-glucose vitually exclusively as the sole soluble monosaccharide, thereby facilitating its ease of purification.

The second variation, whose flow scheme is given in Figure 2, is similar to the sequential acid-enzyme hydrolysis process described above, except that acid hydrolysis is conducted at a temperature less than 80°. The reason for this rests on the observation that acid hydrolysis of corn kernel hulls at lower temperature affords a mixture rich in the pentoses, and with relatively low glucose content, and hence affords a selectivity unattainable at higher temperatures. As stated above, in this embodiment, acid hydrolysis is conducted at a temperature less than 80°C, preferably in the range from 35 to 70°C, and more particularly in the range from 40 to 60°C. The acids which may be used, and the concentration at which they are used, is the same as that described above. Similarly, subsequent treatment of the acid hydrolyzate with a cellulose-degrading enzyme is the same as that previously described.

As shown in Figure 2, the acid hydrolysate can be separated into a liquid portion and a solid portion. As previously mentioned, separation may be by any convenient means, such as membrane separation or filtration, and simple filtration is preferred. The liquid portion consists mainly of monosaccharides, chiefly D-xylose and L-arabinose, as well as some soluble polysaccharides. The solid portion is then resuspended and subjected to enzymatic hydrolysis by a cellulose-degrading enzyme. The resulting hydrolysate contains chiefly D-glucose as its sole monosaccharide.

It is readily seen that this solid separation affords two quite distinct product streams. The liquid portion from separation of the acid hydrolysate contains chiefly pentoses, with some soluble polysaccharides, whereas the enzymatic hydrolysate contains chiefly glucose as its monosaccharide. It thus has the advantages of affording a facile separation between pentoses, on the one hand, and D-glucose, on the other hand. However, it also needs to be recognized that when the solid separation step is practiced, the total glucose yield is reduced, probably because some polysaccharides remain dissolved in the liquid portion of the separated hydrolysate stream.

In yet another embodiment, represented by Figure 3, the corn kernel hulls are mixed with a dilute solution of a strong base at a temperature between 10 and 40°C, and then sequentially hydrolyzed first by a cellulose-degrading enzyme and thereafter by a strong acid, and recovering the resulting hydrolyzate. The purpose of the base pretreatment is to reduce the crystallinity of cellulose, so that it becomes more susceptible to enzymatic hydrolysis.

It will be appreciated that the base pretreatment is unusually mild in comparison to similar treatments for lignocellulosic materials. The identity of the base is unimportant, and such bases as alkali metal hydroxides and carbonates frequently will be employed for convenience. Sodium hydroxide, potassium hydroxide, lithium hydroxide, sodium carbonate, potassium carbonate, lithium carbonate, and mixtures thereof, are examples of suitable bases, which will generally be used at a concentration from 0.5 to 5%, and more preferably at a concentration between 0.5 and 3%. The base pretreatment is conducted at a temperature between 10 and 40°C, preferably between 15 and 30°C, for a time sufficient to achieve its stated purpose. Base pretreatment time will vary with such factors as contact efficiency between the corn kernel hulls and base, temperature, concentration of base, and so forth, but typically will be in the range from 15 to 180 minutes.

The material resulting from base pretreatment, without any filtration, is subjected to hydrolysis using a cellulose-degrading enzyme under conditions previously described. The enzymatic hydrolysate is then hydrolyzed with strong acid as described above, to afford the final product stream.

As in the other two embodiments, this one also has a mode of operation in which the enzymatic hydrolyzate is separated into a liquid portion and a solid portion. The liquid portion, which can be obtained by such separation methods as membrane separation and simple filtration, contains D-glucose as virtually the exclusive monosaccharide and some soluble polysaccharides. Consequently, use of this separation step affords a relatively pure D-glucose stream.

The solid portion is thereafter subjected to hydrolysis with a strong acid, with the acid hydrolysate containing chiefly D-glucose, D-xylose, and L-arabinose. However, because there are some dissolved polysaccharides in the liquid portion of the separation stage the total yield of monosaccharides from this subvariant is less than that where no separation is applied to the enzymatic hydrolysate, and may not be advantageous where yield maximization is important.

## EXAMPLE 1

A 15g sample of corn kernel hulls, obtained as the "expeller output" from a major corn milling operator, was mixed with 200 ml of 7% sulphuric acid for 5.5 to 6.0 hrs. at varying temperatures. The reaction mixture then was cooled, and filtered, and the filter cake was washed with 100 ml of water. The filter cake represented about 25% by weight of starting material. The filtrate was analyzed for glucose, xylose, and arabinose by HPLC, with the results summarized in Table 1 below.

Table 1

| Effect of Temperature in Acid Hydrolysis | | | | |
|---|---|---|---|---|
| Time, hr.<br>T, °C | 5.5<br>100 | 6<br>85 | 6<br>70 | 5.5<br>60 |
| Wt. % Sample Hydrolyzed to: | | | | |
| Glucose | 29.8 | 24.9 | 12.3 | 3.3 |
| Xylose | 14.8 | 13.6 | 14.4 | 8.3 |
| Arabinose | 10.3 | 9.0 | 10.3 | 11.6 |

These results show that the extent of glucose formation is quite temperature-dependent. At temperatures between 70 and 100°C, both xylose and arabinose content appear unaffected by temperature. At 60°C and below, the xylose yield also decreases.

**EXAMPLE 2**

Corn kernel hulls were hydrolyzed in 7% sulphuric acid at 100°C for 5.5 hrs. In Run A, the acid hydrolyzate was filtered and the filter cake was washed, with the washings added to the filtrate, dried, then resuspended in an acetate buffer, 0.1 molar, at pH 4.5, and hydrolyzed with 0.4 wt % cellulase for 24 hrs. at 45°C. In Run B the acid hydrolyzate was filtered, the filter cake was washed with the washings added to the filtrate, and the filter cake was then suspended in an acetate buffer without previous drying. The suspension was then enzymatically hydrolyzed as in A. In Run C, the acid hydrolysate was not filtered, but instead was adjusted to pH 4.5, acetate buffer was added as previously described and the mixture was enzymatically digested. The results are summarized in Table 2 below.

Table 2

| Acid Hydrolysis at Elevated Temperature | | | | | | |
|---|---|---|---|---|---|---|
| Run | Material | Enzyme Glucose | Acid Glucose | Xylose | Arabinose | Total Glucose |
| A | filtrate from acid hydrolyzate cellulase hydrolyzate | 1.2 | 26.9 | 14.9 | 10.5 | 28.1 |
| B | filtrate from acid hydrolyzate cellulase hydrolyzate | 14.0 | 25.0(25.5)[a] | 18.6(14.8) | 9.7(10.6) | 39.5 |
| C | acid hydrolyzate cellulase | 8.8[b] | 23.5 | 13.7 | 9.8 | 32.3 |

a. Figures in parenthesis represent duplicate run.
b. Figures represent incremental glucose production.
c. Figures for monosaccharides are weight percent based on total corn hulls used.

These results show that for maximization of glucose content, filtration of the acid hydrolysate is required, but the filter cake should not be dried before enzymatic hydrolysis.

5

EP 0 344 371 A1

## EXAMPLE 3

A mixture of 14.9 g of dried corn kernel hulls and 200 ml of 7% sulphuric acid were heated at 60°C for 5.5 hours. The cooled mixture was filtered, and the cake was washed with 100 ml of water. Analysis of the combined washings and filtrate showed they contained 2.5% glucose, 8.4% xylose, and 10.1% arabinose, based on the initial weight of hulls.

The filter cake was suspended in 250 ml of distilled water and the pH was adjusted to 4.8. After acetate buffer at pH 4.8 (1 molar, 30 ml) and 1.2 g of cellulase were added, the volume was adjusted to 300 ml. After the mixture was incubated on a shaker at 45°C for 24 hours, it was filtered, the filter cake amounting to 9.8% by weight of the hulls. Analysis of the filtrate showed it contained 17.4% glucose and under 1% of other monosaccharides, based on initial hull weight.

## EXAMPLE 4

Samples of corn kernel hulls were mixed with a solution of 1.5% sodium hydroxide (75 ml base per 5 g hulls) at ambient temperature for 90 minutes. The mixture was adjusted to pH 4.5, acetate buffer at the same pH was added to 0.1 molar concentration, and the mixture was digested with 0.4% (W/V) cellulase for 24 hours at 45°C. In Run A, the enzyme hydrolyzate was filtered and washed, with the washings added to the filtrate, and the filter cake was subjected to acid hydrolysis at 100°C, as described in Example 3. In Run B, the enzyme hydrolysate was not filtered, but the entire hydrolyzate was subjected to acid hydrolysis as previously described. Results are tabulated below, with monosaccharides given in weight percent, based on hulls used.

Table 3

| Mild Base Pretreatment of Corn Kernel Hulls | | | | | | |
|---|---|---|---|---|---|---|
| Run | Material | Enzyme Glucose | Acid | Xylose | Arabinose | Total Glucose |
| A | filtrate from acid hydrolyzate acid hydrolyzate | 34 | 0.6 | 1.3 | 0.7 | 34.6 |
| B | enzyme hydrolyzate acid hydrolyzate[a] | 34 | 5 | 17.5 | 10.5 | 39 |

a. figures represent incremental monosaccharide production.

These results show that a rather pure glucose stream can be obtained by enzymatic hydrolysis of a mildly base-treated corn hull feedstock. They also show that the enzyme-treated material should not be filtered before acid hydrolysis for maximum monosaccharide formation. Other data also demonstrate that the initial base-digested material should not be filtered before enzymatic hydrolysis, for if only the filtrate is treated with enzyme, the glucose yield is reduced to 15% from the 34% obtained without filtration.

## Claims

1. A method of producing monosaccharides by hydrolysis of lignocellulosic materials, characterized in that corn kernel hulls are subjected to separate hydrolysis steps with a strong acid and with a cellulose-degrading enzyme.

2. A method according to claim 1 characterized in that the corn kernel hulls are initially hydrolyzed with the strong acid at a temperature of 80 to 110°C, and the acid hydrolyzate is then subjected to the enzyme hydrolysis step.

3. A method according to claim 2 characterized in that the acid hydrolysate is separated into a liquid portion, whose monosaccharides are chiefly D-glucose, D-xylose, and L-arabinose, and a solid portion, the enzyme hydrolysis step being carried out on the solid portion thereby producing an enzymatic hydrolysate containing chiefly D-glucose.

6

4. A method according to claim 1 characterized in that the corn kernel hulls are initially hydrolyzed with the strong acid at a temperature of 35 to 80°C, and the acid hydrolyzate is then subjected to the enzyme hydrolysis step.

5. A method according to claim 4 characterized in that the acid hydrolysate is separated into a liquid portion, whose monosaccharides are chiefly D-xylose and L-arabinose, and a solid portion, the enzyme hydrolysis step, being carried out on the solid portion, thereby producing an enzymatic hydrolyzate containing chiefly D-glucose.

6. A method according to claim 1, characterized by the steps of: mixing the hulls with a dilute solution of a strong base at a temperature between 10 and 40°C, subjecting the base-treated hulls to hydrolysis by the cellulose-degrading enzyme and thereafter subjecting at least a portion of the enzymatic hydrolyzate to the acid hydrolysis step.

7. A method according to claim 6 characterized in that the enzymatic hydrolysate is separated into a liquid portion, whose monosaccharide is chiefly D-glucose, and a solid portion, which is thereafter subjected to the acid hydrolysis step, thereby producing an acid hydrolyzate containing chiefly D-glucose, D-xylose, and L-arabinose.

8. A method according to any one of the preceding claims characterized in that the enzyme is cellulase and the enzymatic reaction is conducted at a temperature between 25 and 55°C and at a pH between 3 and 6.

9. A method according to claim 8 characterized in that the enzymatic hydrolysis is conducted at a temperature between 35 and 55°C.

pH ADJUST

CORN KERNEL HULLS → ACID HYDROLYSIS > 80°C → ENZYMATIC HYDROLYSIS → FILTER → D-GLUCOSE +OTHERS IF NO SEPARATION STAGE

SEPARATION — SOLID

D-GLUCOSE

L-ARABINOSE

D-XYLOSE

*FIG. 1*

pH ADJUST

CORN KERNEL HULLS → ACID HYDROLYSIS < 80°C → ENZYMATIC HYDROLYSIS → GLUCOSE (MONOSACCHARIDES IF NO SEPARATION STAGE)

SEPARATION — SOLID

LIQUID → MONOSACCHARIDES (PRIMARILY PENTOSES) & SOME POLYSACCHARIDES

*FIG. 2*

pH ADJUST

CORN KERNEL HULLS → BASE → ENZYMATIC HYDROLYSIS → ACID HYDROLYSIS → L-ARABINOSE & D-XYLOSE & D-GLUCOSE

SEPARATION — SOLID

GLUCOSE & POLYSACCHARIDES

*FIG. 3*

European Patent Office

EUROPEAN SEARCH REPORT

Application Number

EP 88 30 4960

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 490 677 (INSTITUT FRANCAIS DU PETROLE) <br> * Claims 1-6; page 3, lines 11-30; example 1 * <br> --- | 1,2,3,5 ,8,9 | C 13 K 1/02 <br> C 13 K 13/00 <br> C 12 P 19/14 |
| X | FR-A-2 419 351 (PURDUE) <br> * Claims 1,2,11,12,15,17,18-20; page 9, lines 6-15; example 1 * <br> --- | 1,3,8,9 | |
| A | US-A-4 089 745 (ANTRIM) <br> * Claims 1,2; example * <br> ----- | 1,6,7 | |

| | |
|---|---|
| | TECHNICAL FIELDS SEARCHED (Int. Cl.4) |
| | C 13 K <br> C 12 P |

The present search report has been drawn up for all claims

| Place of search | Date of completion of the search | Examiner |
|---|---|---|
| THE HAGUE | 24-01-1989 | VAN MOER A.M.J. |